# EUROPEAN PATENT APPLICATION

(11) **EP 4 443 001 A1**
(43) Date of publication of application: **09.10.2024**
(21) Application number: 24167286.4
(22) Date of filing: 28.03.2024
(51) Int. Cl.: F04B 43/12

(54) **DEVICE FOR A LINEAR PERISTALTIC PUMP, ROTOR AND LINEAR PERISTALTIC PUMP**

(30) Priority: 06.04.2023 DE 102023108837
(71) Applicant: Gardner Denver Thomas GmbH, 82256 Fürstenfeldbruck (DE)
(72) Inventor: SCHMID, Gunther Erich, 81241 München (DE)
(74) Representative: Murgitroyd & Company

(57) **Abstract**

The invention relates to a device for a linear peristaltic pump (1) for conveying a fluid through a flexible tube (40), with a track or guide- or slideway (100), a rotor (12) for a device (10) and a linear peristaltic pump (1), wherein the device comprises a continuously running track or slideway (100), wherein the track or slideway (100) has, in sections, a circular course (101) with a radius (R) and a midpoint (M). Further, the track or guideway or slideway (100) comprises, first and second subregions, which are preferably curved courses spaced apart from the circular course and configured such that in the radial direction, a first length (L1, L11) is a difference between the radius (R) and a first spacing (110, 111) relative to the circular course and wherein, in the radial direction, a second length (L2, L12) is a sum of the radius (R) and a second spacing (120, 121) relative to the circular course.

## Description

The invention relates to a device for a linear peristaltic pump. The invention further relates to a rotor for a device. Moreover, the invention relates to a linear peristaltic pump.

Peristaltic pumps have been known from the prior art for a long time.

A tube pump, also called a tube-squeezing pump or peristaltic pump, is a positive displacement pump in which the medium to be conveyed is pushed through a flexible tube through external mechanical deformation thereof. Rolling pumps, roller pumps or tube-squeezing pumps are preferably known. Rollers or sliding blocks of a rotor impel a medium, preferably fluid, which is to be pumped and which is present in a tube at the occlusion locations produced by the contact of the rollers or sliding blocks on the tube.

US 2009/240201 A1 describes a peristaltic pump with a plurality of pushrods, wherein the pushrods are set up to periodically squeeze a flexible infusion tube. Furthermore, tube pumps are known in which changing tubes proves to be complicated.

GB 2 230 301 A relates to a known peristaltic pump with a rotor having two or more rollers mounted thereon via flexible arms. The rollers exerting pressure upon a flexible tube to forward a liquid. Such rollers and similar rotors having spring loaded arms have the following disadvantages: The forces exerted on the tube depends on the springs used and cannot be adapted, except the design of the system allows that the distance between a part supporting the flexible tube and the rotational axis of the rotor is varied. Consequently, the amount of liquid product forwarded in the tube will also be varied.

Another disadvantage of rotors using flexible arms with rollers or spring-loaded rollers is that the forces act at full strength on the rotor shaft and must be absorbed by the shaft of the motor or corresponding bearings.

Another disadvantage for common peristaltic pumps that they are not adjustable and thereby the force curve in the linear section rises from a minimum value to a maximum and then falls again. Thus the object of continuous pressure build-up cannot be met. Accordingly, it is one technical object to provide a continuous pressure during the full length, when the rollers contact the tube.

A further disadvantage of the pumps from the prior art is that they have a complex structure which has a disadvantageous effect in the event of maintenance and/or repair.

The problem addressed by the present invention is to overcome the disadvantages from the prior art. Furthermore, it is intended to provide alternative devices, rotors and linear peristaltic pumps.

The problem is solved through a device for a linear peristaltic pump for conveying a fluid through a flexible tube. The device comprises a continuously running track or guideway or slideway, wherein the running track or slide- / guideway has, in sections, a circular course with a radius and a midpoint, wherein the track or slideway further comprises
a) a first subregion with a linear or curved course which runs spaced apart relative to the circular course and has, in the radial direction, a first maximum spacing, and
b) a second subregion with a curved course, wherein the second subregion is arranged opposite the first subregion, and wherein the second subregion runs spaced apart relative to the circular course and, in the radial direction, has a second maximum spacing,
c) wherein the first and second maximum spacings are of identical size.

In other words, the first subregion and the second subregion represent amendments or deviations from the circular course or of the circular course.

The linear or curved course of the first subregion, which runs spaced apart relative to the circular course results in a plurality of first spacings relative to the circular course, wherein one of the plurality of first spacing is a first maximum spacing. Preferably there is only one first maximum spacing. Similarly, the curved course of the second subregion has a plurality of second spacings comprising one second maximum spacing.

As a result of the provision of the device with the defined track/guideway or slideway, it is possible to use a rotor which can be inserted into the device. During operation of the peristaltic pump the squeezing means of the inserted rotor are guided along the guideway.

A track or guideway can be understood to be a roller track. A slideway can be understood to be a sliding block track.

The track, guideway or slideway is preferably set up to guide the rotor or squeezing means of a rotor. A squeezing means may preferably be a means for occluding, pressing on, clamping, preferably clamping-off, pressing-off or pressing-together or compressing a tube. In particular, the squeezing means may be a roller or a sliding block. A roller may also be understood to be a rolling body, a rollable body, a rolling bearing or the like.

Furthermore, it is possible to squeeze, occlude, clamp, preferably clamp off, press off or press together or compress, preferably in the region of the first subregion, by means of the rotor, a flexible tube which is inserted into the device, in order to convey fluid through a tube in a defined manner.

A device can be understood to be a control link, a stator or a housing. A linear peristaltic pump is also known as a roller pump.

Preferably, in the linear peristaltic pump, the tube is introduced or inlaid into the device without curving the longitudinal axis of the tube using an opposed running surface. In other words, the tube is introduced or inlaid into the peristaltic pump or into the device of the pump in an uncurved or untwisted manner. Thus, according to this preferred embodiment the linear peristaltic pump has a rectilinear opposed running surface and the first subregion of the track or guide- or slideway is also rectilinear. Due to the special geometry of the second subregion with a curved course, wherein the second subregion is arranged opposite the first rectilinear subregion, the forces acting on the tube depend on how the opposed running surface is adjusted. Said opposed running surface of the tube can be spring-loaded, wherein the spring preload of the opposed surface can be varied to change the forces.

Since in contrast to common roller pumps the rotor members do not use springs or flexible parts, it is an advantage of the special design of the second subregion that the forces can be directly introduced into the housing and not into the bearing which holds the rotor.

A device is thus provided which has, compared to other linear solutions, a simple structure and which is less complex to maintain. Especially the at least one shaft of the motor for rotating the rotor is significantly less or not stressed in the radial direction. In this way the rotor or parts thereof as arms can be mounted in the device in a spring-free manner or without flexible elements. In contrast to the prior art the squeezing means or rollers, which oppose each other, can be connected with a support of a constant elongation. In this way the opposing squeezing means have a fixed spacing and are rigidly coupled.

The track or guideway or slideway has, in sections, a circular course with a radius and a midpoint. Thus portions or sections of a circular course are formed. For instance, these sections may be formed by two arcs. The arcs are preferably spaced apart from one another. In other words, they maybe in a preferred embodiment symmetrical to each other and have a defined spacing, preferably a fixed spacing with regard to the symmetrical axis.

"Continuous" can be understood to be a course which is enduring, seamlessly connected, uniformly continuing, further-continuing and/or continual.

A curved course can be understood to be a domed course. The domed course may preferably be an ellipsoid dome or oblate ellipsoid. The curved course is preferably symmetrical. The curved course may preferably follow a mathematical 1st order function or (1 +n) order function, wherein n=>1. The curved course may have a vertex and be configured in an ellipsis shape, a parabola shape, a catenary shape, preferably as a catenary, catenoid or funicular curve.

In a preferred embodiment, the first subregion is arranged inside an orbit of the circular course, with the second subregion being arranged outside the orbit of the circular course. As a result, a continuous course of the track or guide- / slideway can be produced.

In a further preferred embodiment, in the radial direction, a first length is a difference between the radius and the first spacing. Furthermore, in the radial direction, a second length is a sum of the radius and the second spacing. In other words, the first length is smaller than the second length. That is to say during operation of the peristaltic pump with a rotor the first and the second lengths may be a variable length dependent on the angle of rotation of the rotor.

In this way a constant squeezing force upon the tube can be provided by the squeezing rollers or other squeezing means. The first and seconds lengths are varied as a function of the angle of the radius of the circular course and thus are during operation dependent of the angle of rotation of the rotor such that the spacing between the centers of the opposed squeezing means as for example rollers stay the same independent of the angle of rotation of the rotor. Accordingly the force applied to the tube, when contacting the tube with a squeezing mean or element such as a roller, can only be varied if the opposed running surface of the tube is pressed more or less against the tube. The pressing of the opposed running surface toward the tube can either be done by using a spring mechanism or other mechanical means to adjust the distance of the opposed running surface with relation to the tube.

In yet another preferred embodiment, the first length defines a first point on the first subregion and the second length defines a second point on the second subregion. Thus, the first point is closer to the midpoint M than the second point.

In a further preferred embodiment, the first maximum spacing is located in the middle of the first subregion and the second maximum spacing is located in the center or at the symmetrical axis of the second subregion. As a result, a continuous course of the track or guide-/ slideway can be produced. "In the middle" can be understood to mean "in the center". In other words, the first point is arranged in the middle on the first subregion and the second point is arranged in the middle on the second subregion.

In a preferred embodiment, two arcs of the circular course are arranged opposite one another. As a result, the course of the track or guide-/ slideway can be optimized.

In a further preferred embodiment, both ends of the first subregion and of the second subregion are connected to one section, subsection or arc of the circular course each. As a result, a continuously running track or guideway or slideway can be produced in a simple manner. A section may be understood to be an arc.

The first subregion with the linear course preferably has, at the end, transition regions to the circular course. As a result, a continuous transition of the linear course to the circular course can occur. Transition regions are preferably configured or arranged in the region or at transitions between the circular course and the first subregion and/or the second subregion. In other words, transition regions can be configured at the transitions between the first and/or second subregion and the circular course. A transition region can be a bend, dome, straight line or a curved course, as described above.

In a further embodiment, the second subregion is configured to be convex. In other words, the sum of the first maximum value is always larger than half the diameter (radius R) of the circular course.

In a further preferred embodiment, the curved course of the first subregion is
- configured to be arched toward the midpoint, or
- configured to be arched away from the midpoint (M).

As a further alternative, the first subregion is configured to be linear, as is mentioned above. With these embodiments, it is possible to configure the track for the rotor in a different manner. The course of the second subregion should be adapted in accordance with the configuration of the course of the first subregion, in order to guarantee the function of a rotor.

In an even further preferred embodiment, the device has a groove for accommodating a tube. The groove can have an aperture in the region of the first subsection. Alternatively, the first subsection can have an interruption. The device can have a closure arranged opposite the groove, aperture and/or interruption or have a cover in order to avoid the tube escaping out of the device. Furthermore, a tube-clamping device can be used to secure the tube.

The groove thus serves to accommodate a flexible tube. When a tube is laid into the groove, the tube protrudes preferably at least partially through the aperture into the device. By means of a rotor, the tube can be squeezed, clamped, preferably clamped off, pressed off or pressed together or compressed in order to convey fluid through the flexible tube in a defined manner. Since the tube protrudes towards the inside of the device, i.e. inwards, in the region of the groove, it can be squeezed, clamped, preferably clamped off, pressed off or pressed together when a squeezing means contacts the tube.

In a particularly preferred embodiment, the device is set up to squeeze, clamp, occlude, preferably clamp off, press off or press together or compress the tube by means of at least one squeezing means, preferably a roller or sliding block of a rotor which is rotatably mounted in the device. A roller may also be understood to be a rolling body.

"At least" can also be understood to mean "at a minimum".

Furthermore, the problem is solved through a rotor for a device, preferably as described above, a linear peristaltic pump for conveying a fluid through a flexible tube, having
- at least one first pair of squeezing means having a first and a second squeezing means,
- at least one squeezing means support for accommodating the first and second squeezing means, and
- at least one cam for guiding and/or accommodating the at least one squeezing means support.

A pair of squeezing means can be understood to be a first and second roller or a first and second sliding block. As mentioned above, a squeezing means may be a means for pressing on, clamping, preferably clamping-off, pressing-off or pressing-together or compressing a tube.

A squeezing means support can be a roller support or a sliding block support. A squeezing means support can be understood to be a guiding means or a squeezing means guiding means.

There are preferably two squeezing means supports arranged in the rotor, each having two squeezing means. The cam preferably has a shaft. Particularly preferably, the cam is set up to be actuated by means of a motor, i.e. to be caused to rotate.

A rotor is thus provided which has a simple structure and which is inexpensive to manufacture. As a result of the multipart structure, it is easy to replace components which need to be exchanged.

In a preferred embodiment, the cam has slots, cavities or recesses, wherein the at least one squeezing means support is displaceably arranged in a slot, cavity or recess. The cam can be formed in a one-piece or two-piece manner.

In a preferred alternative embodiment, the cam comprises two cam members arranged parallel to one another and spaced apart from one another, wherein the cam members have slots, cavities or recesses for guiding the squeezing means, and wherein the at least one squeezing means support is displaceably arranged between the cam members.

Each squeezing means preferably has a shaft. Alternatively, each squeezing means has, on both sides, at the front, in each case a protruding region, a pin or peg. The shaft, the protruding regions or the pins are preferably displaceably arranged in the slots, cavities or recesses.

The rotor is preferably configured to displace the at least one squeezing means support relative to the cam. Furthermore, the rotor is set up to squeeze, clamp, preferably occlude, clamp off, press off or press together or compress a tube which is laid into a groove of the device and which then preferably at least partially protrudes through the aperture into the device, in order to convey fluid through the flexible tube in a defined manner.

In a further preferred embodiment, one squeezing means in each case is rotatably arranged on the ends of each squeezing means support.

The squeezing means preferably comprises a section which protrudes out of the device.

Two squeezing means are preferably rotatably arranged on the end on at least a second squeezing means support. At least two additional squeezing means are preferably rotatably arranged on the end on at least a further squeezing means support.

Particularly preferably, each squeezing means is rotatably and displaceably arranged on the end on two squeezing means supports which are parallel to one another.

In a further preferred embodiment, a first longitudinal axis of the first squeezing means support and a second longitudinal axis of a second squeezing means support are arranged perpendicular to one another.

A longitudinal axis can be understood to be a centre axis or center longitudinal axis. The longitudinal axes of three squeezing means supports are preferably arranged at an angle of 60° to one another. The longitudinal axes of four squeezing means supports are preferably arranged at an angle of 45° to one another.

In a particularly preferred embodiment, the at least one squeezing means support is set up so as to be displaced relative to the cam.

In yet another preferred embodiment, in the event of two or more squeezing means supports, the squeezing means supports are set up to be displaced relative to the cam and/or relative to one another.

All components of the rotor are preferably mounted in the device in a spring-free manner or are displaceable. All components of the rotor are preferably operatively connected to one another.

Furthermore, the problem is solved through a linear peristaltic pump comprising at least a device, preferably as described above, and a rotor, preferably as described above. Said device comprises a continuously running track or guideway or slideway, wherein the track or guide-/ slideway has, in sections, a circular course with a radius (R) and a midpoint, wherein the track or slideway further comprises
a) a first subregion with a linear or curved course which runs spaced apart relative to the circular course and has, in the radial direction, a plurality of first spacings (111, 110) comprising a first maximum spacing (110), and
b) a second subregion (103) with a curved course, wherein the second subregion (103) is arranged opposite the first subregion (102), and wherein the second subregion (103) runs spaced apart relative to the circular course (101) and, in the radial direction, has a pluraltiy of second spacings (120, 121) comprising a second maximum spacing (120),
c) wherein the first and second maximum spacings (110, 120) are of identical size.

In a preferred embodiment, the linear or curved course of the first subregion and the curved course of the second subregion are configured such that in the radial direction, a first length (L1, L11) is a difference between the radius (R) and a first spacing (110, 111) relative to the circular course and wherein, in the radial direction, a second length (L2, L12) is a sum of the radius (R) and a second spacing (120, 121) relative to the circular course.

A linear peristaltic pump is thus provided which has a simple structure and which is inexpensive to manufacture. As a result of the multipart structure, components which need to be exchanged can be easily replaced. In contrast to common roller pumps, wherein the rotor members use springs or flexible parts and produce stress on a shaft, the special design of the first and second subregions of the device of the peristaltic pump leads to the advantage that forces can be directly introduced into the housing of the device and not into shaft of the motor rotating the rotor.

When a tube is laid into the groove, the tube protrudes preferably at least partially through the aperture into the device. By means of a rotor, the tube can then be squeezed, clamped, preferably clamped off, pressed off or pressed together or compressed in order to convey fluid through the flexible tube in a defined manner.

In a preferred embodiment, the linear peristaltic pump comprises at least two devices with one rotor each. The two devices are preferably arranged relative to one another such that their grooves for accommodating a tube are situated opposite one another. The tube can preferably be arranged in the groove jointly formed by both devices.

In a further preferred embodiment, the at least one first squeezing means support is set up to be displaced relative to the track or guide-/slideway. In this case, the squeezing means are set up to contact the track or guide-/slideway.

A displacement of the at least one squeezing means support is preferably accompanied by a rotation of the rotor.

In a further preferred embodiment, the linear peristaltic pump furthermore comprises a drive for driving the rotor. Thus, the rotor can be driven in a simple manner.

In yet another preferred embodiment, the track or guideway is configured, at the edge, as a toothed rack or as a toothing and the at least one squeezing means has, at the edge, a region configured as a sprocket, wherein the toothed rack or the toothing meshes with the sprockets of the squeezing means.

In a particularly preferred embodiment, the linear peristaltic pump is set up to squeeze, occlude, clamp, preferably clamp off, press off or press together or compress, by means of the rotor, a flexible tube which is laid into a groove of the device and which protrudes into the aperture of the groove, in order to convey fluid through the flexible tube.

The squeezing means are preferably set up to use the squeezed tube as a track. This can be the case if the tube, in the squeezed state, i.e. when at least one squeezing means is in squeezing means contact with the tube, still protrudes into the device. Preferably, the device and the rotors are dimensioned such that they allow tolerances, in order to guarantee rotation of the rotor in the device and a squeezing of the tube.

Particularly preferably, the toothed rack or the toothing is arranged on at least one edge section of the device.

The invention will be explained in more detail below with reference to exemplary embodiments. Various features and advantages of the invention will be set forth in part in the following description of Figures referring to various examples of the invention. In this context it is noted that the dimensions shown in all Figures of this disclosure are not to scale and any directions as e.g. vertical are not limiting. The illustrations are simplified and not in each Figure all components are indicated with reference numerals and like reference numerals may be carried forward. In this description, reference is made to the accompanying Figures, in which drawings:
- Fig. 1: shows a schematic depiction of a device,
- Fig. 2: shows a section A-A through the device from Fig. 1,
- Fig. 3: shows the section A-A from Fig 1;
- Fig. 4: shows an alternative configuration of a track guide,
- Fig. 5: shows an additional alternative configuration of a track guide,
- Fig. 6: shows a schematic depiction of components of a rotor,
- Fig. 7: shows the components from Fig. 6 with rollers,
- Fig. 8: shows a schematic depiction of an additional component of a rotor,
- Fig. 9: shows a schematic depiction of a rotor in the assembled state,
- Fig. 10: shows a schematic depiction of an alternative rotor,
- Fig. 11: is a view of the rotor from Fig. 10;
- Fig. 12: shows a schematic depiction of a linear peristaltic pump,
- Fig. 13: shows a further schematic depiction of the linear peristaltic pump from Fig. 12,
- Fig. 14: shows an exploded depiction of parts of the linear peristaltic pump,
- Fig. 15: shows a lateral view onto the linear peristaltic pump from Fig. 13,
- Fig. 16: shows a section B-B according to Fig. 15 with a rotor in a first position,
- Fig. 17: shows a section B-B according to Fig. 15 with the rotor in a second position,
- Fig. 18: shows a section B-B according to Fig. 15 with the rotor in a third position,
- Fig. 19: shows a section B-B according to Fig. 15 with the rotor in a fourth position,
- Fig. 20: shows a section B-B according to Fig. 15 with an alternatively configured rotor in an additional position,
- Fig. 21: shows a partial view of the device from Fig. 1 in an alternative configuration,
- Fig. 22: shows a section through an alternatively configured linear peristaltic pump with two rotors in a first position, and
- Fig. 23: shows an additional section through the alternatively configured linear peristaltic pump from Fig. 22 with the two rotors in a second position.

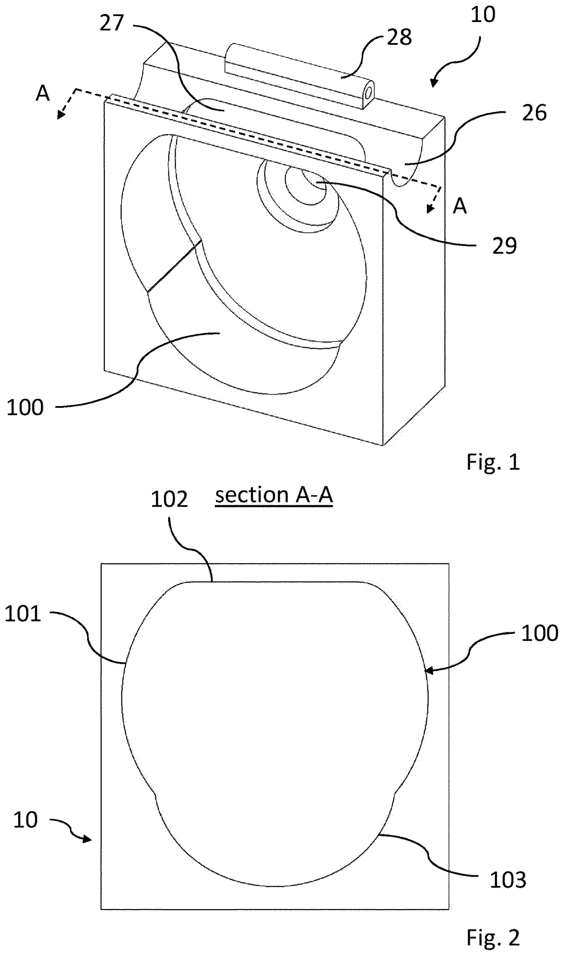

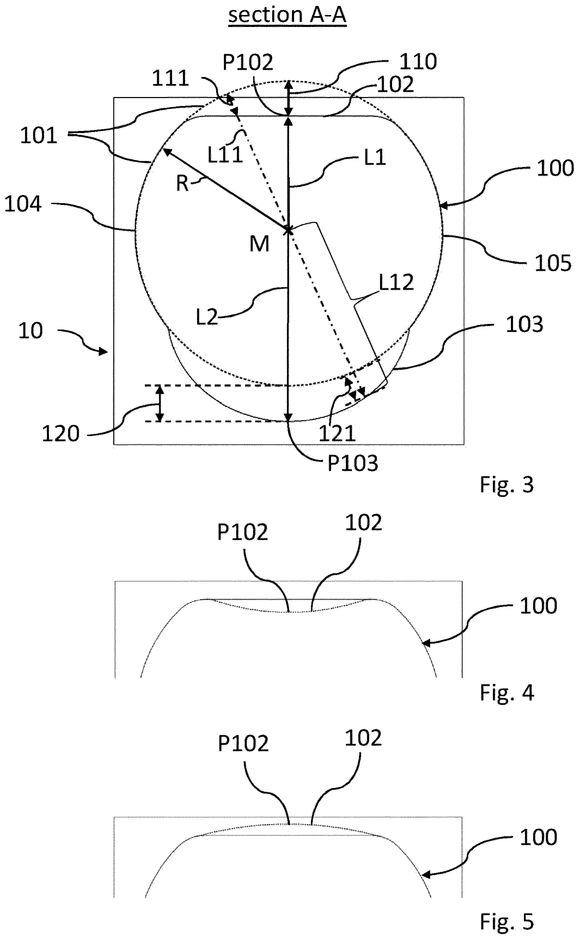

Fig. 1 shows a schematic depiction of a device 10. The device 10 has a track or guideway or slideway configured as a roller track 100, a groove 26, an aperture 27, a connector 28 and, at the rear wall, an aperture for connecting a drive for a rotor (not shown). The device 10 is for a linear peristaltic pump (not shown) for conveying a fluid through a flexible tube (not shown), which can be laid into the groove 26. The groove 26 has the aperture 27 which is located in the roller track 100 in an upper region of the device 10. The groove 26 runs in sections parallel to a first subregion 102, as also shown in Figs. 2 and 3. The groove 26 is set up such that a tube laid into the groove 26 can protrude into the aperture 27.

The connector 28 serves to accommodate a closure or a cover. The device 10 can be understood to be a control link, stator or housing.

The device 10 is set up to squeeze, clamp, preferably occlude, clamp off, press off or press together or compress said tube, which protrudes from the aperture 27, in the event of contact with the roller by means of at least one squeezing means of a rotor which is rotatably mounted in the device 10. By means of a rotor, the open tube cross-section can be reduced such that fluid is conveyed through the flexible tube in a defined manner.

Fig. 2 and Fig. 3 show a section A-A through the device 10 from Fig. 1. The roller track 100 is configured to run continuously. The roller track 100 has sections of a circular course 101, wherein two arcs or sections (104 and 105) of the circular course 101 are arranged opposite one another. The sections 104 and 105 are symmetrical to each other with relation to a vertical symmetrical axis along the length L1. The circular course 101 has a radius R and a midpoint M and is shown with a dotted line. The roller track 100 further comprises a first subregion 102 with a, preferably approximately, linear or curved course which runs spaced apart relative to the circular course 101 (see dotted line) and has, in the radial direction, a first maximum spacing 110. The roller track 100 further comprises second subregion 103 with a curved course, wherein the second subregion 103 is arranged opposite the first subregion 102, and wherein the second subregion 103 runs spaced apart relative to the circular course 101 and, in the radial direction, has a second maximum spacing 120.

The first subregion 102 is arranged inside an orbit of the circular course 101. The second subregion 103 is arranged outside the orbit of the circular course 101. The circular sections 103 and 105 are arranged on or part of the circular course. In the radial direction, starting from the midpoint M, a first length L1 corresponds to the difference between the radius R and the first spacing 110. In the radial direction, starting from the midpoint M, a second length L2 corresponds to the sum of the radius R and the second spacing 120. The first length L1 is smaller than the second length L2. In other words, the sum of the first length L1 and the second length L2 corresponds to double the radius R or the length of the diameter (2R) of the circle. Thus, the first point P102 is closer to the midpoint M than the second point P103.

The first length L1 defines a first point P102 on the first subregion 102. The length L1 can be understood as the minimum spacing from the midpoint M to point P102. The second length L2 defines a second point P103 on the second subregion 103. The length L2 can be understood as the maximum spacing from the midpoint M to point P103.

A first maximum spacing 110 and second maximum spacing 120 are the same size or elongation. The first maximum spacing 110 is arranged in the middle of the first subregion 102. The second maximum spacing 120 is in the middle of the second subregion 103. In other words, the first point P102 is in the middle on the first subregion 102 and the second point P103 is in the middle on the second subregion 103.

The first subregion 102 and the second subregion 103 are connected to the circular course 101 configured in sections. In other words, the first and second subregions 102, 103 are attached to the arcs. Transition regions can be configured in the transitions between the circular course 101 and the first subregion 102. Transition regions can also be configured in the transitions of the second subregion 103.

Furthermore Fig. 3 shows a further spacing 111 in the radial direction from the first subregion 102 to the circular course 101, wherein said further spacing is on the side, where the tube is received during operation of the linear peristaltic pump. In the radial direction, starting from the midpoint M and pointing to the double arrow of the further spacing 111, a first length L11 corresponds to the difference between the radius R and the further spacing 111. In the radial direction, between the second subregion 103 and the midpoint M, a second length L12 corresponds to the sum of the radius R and the further spacing 121, which spacing is opposite of the spacing 111 on the tube receiving side.

The first length L11 is smaller than the second length L22. In other words, the sum of the first length L11 and the second length L12 corresponds to double the radius R and thus corresponds to the diameter of the circle with midpoint M or circular course 101. It is noted that the further spacing 121 opposite of the spacing on the tube receiving side is smaller than the second spacing 120. That is to say the second subregion 103 increases the spacing from the further spacing 121 to the spacing 120 (both spacings 121 and 120 are shown as double arrows between parallel dashed lines to better illustrate the distance/spacings).

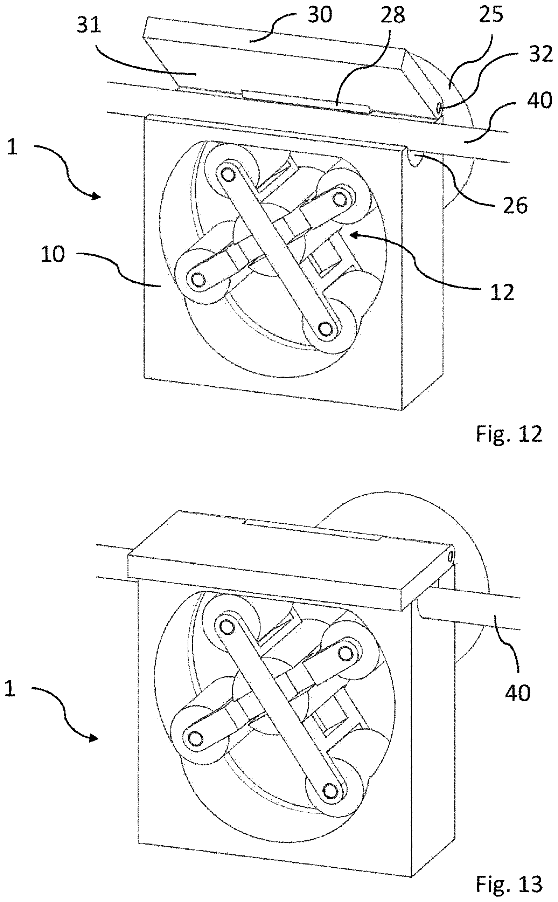

If during operation of the peristaltic pump a rotor 12 with rollers 15 is placed into the device 10 (as shown inter alias in Fig. 12, Fig. 18 or Fig. 20) such that the outer circumference of the rollers 15 is in contact with the track or guideway, the maximum elongation of the rollers 15 and the corresponding support corresponds to the diameter of the circular course 101. Thus, there is a fixed coupling of the squeezing means provided and no elastic elements or springs are required in any part of the rotor. This has the advantage that the forces are directly transferred into the housing of the device 10 of the peristaltic pump.

Fig. 4 shows a first alternative configuration of the first subregion 102. The curved course of the first subregion 102 is configured to be arched toward the midpoint M. The second subregion (not shown) can be configured to be convex.

Fig. 5 shows a second alternative configuration of the first subregion 102. The curved course of the first subregion 102 is configured to be arched away from the midpoint M. The second subregion (not shown) can be configured to be convex.

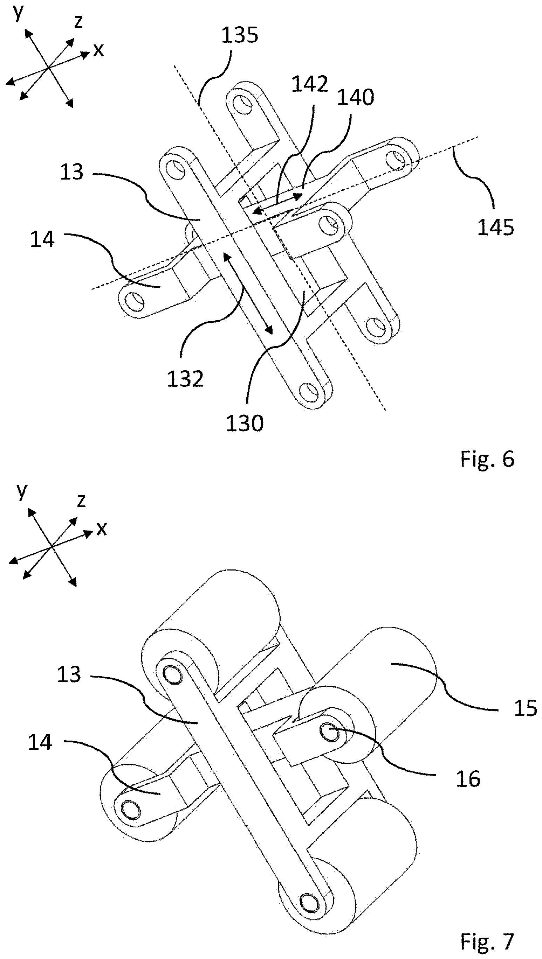

Fig. 6 to Fig. 11 show an x-y-z coordinate system, in order to better describe the spatial conditions or orientations.

Fig. 6 shows a schematic depiction of two components 13, 14 of a rotor. The components 13, 14 can be understood to be squeezing means supports configured as roller supports. A first component 13 has an aperture 130. A second component 14 has a bar 140 or middle piece. The bar 140 of the second component 14 is displaceably arranged in the aperture 130 of the first component 13, preferably in a contact-free manner.

The aperture and the bar are dimensioned such that the first component 13 is displaceable in a y-direction. The displacement of the first component 13 in the y-direction can occur independently of the second component 14, see first direction of movement 132. The aperture 130 and the bar 140 are dimensioned such that the second component 14 is displaceable in an x-direction. The displacement of the second component 14 in the x-direction can occur independently of the first component 13, see second direction of movement 142.

An accommodating possibility for rollers or rolling bodies is arranged at a first and a second end of the first component 13. An accommodating possibility for rollers or rolling bodies is also arranged at a first and a second end of the second component 14.

Fig. 7 shows the two components 13, 14 from Fig. 6 with four squeezing means configured as rollers 15. Alternatively, sliding blocks (not shown) can be used instead of the rollers 15. At the ends, i.e. on the first and second ends of the first component 13 and, at the ends, on the first and second ends of the second component 14, there is arranged one roller 15 in each case. Two of the rollers 15 are rotatably mounted on the first component 13 by means of shafts 16. Two further rollers 15 are rotatably mounted on the second component 13 by means of shafts 16. As an alternative to the shafts, bolts, pins or the like can also be used.

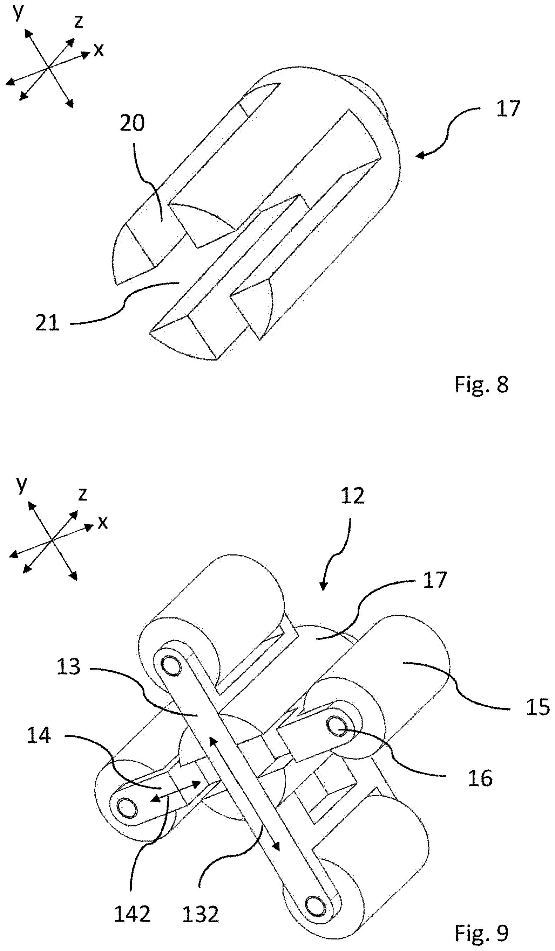

Fig. 8 shows a schematic depiction of a further component for a rotor. The further component is a cam 17. The cam 17 is configured as one piece. The cam 17 has slots 20, 21. A first slot 20 extends in a y-z plane. A second slot 21 extends in an x-z plane. Alternatively, the slots can also be designed as cavities or recesses.

Fig. 9 shows a schematic depiction of a rotor 12 in the fully assembled state without a drive device (motor). In this case, the components, as shown in Fig. 7, are arranged in the cam 17 or introduced into the cam 17, as shown in Fig. 7.

The rotor 12 is envisaged for a device 10 of a linear peristaltic pump for conveying a fluid through a flexible tube, as described above. The rotor 12 has the first roller support 13 with a first pair of rollers and the second roller support 14 with the second pair of rollers. The cam 17 is set up to accommodate the first roller support 13 and the second roller support 14 and to guide or displace the roller supports 13, 14 when the rotor 12 is in operation. A displacement of the roller supports 13, 14 and thus of the rollers 15 takes place along the directions of movement 132, 142.

A first longitudinal axis 135 of the first roller support 13 and a second longitudinal axis 145 of the second roller support 14, as shown in Fig. 6, are arranged perpendicular to one another in the cam 17. The roller supports 13, 14 are displaceably arranged in the cam 17.

The roller supports 13, 14 and the rollers 15 are set up to be displaced relative to one another. A displacement occurs in a linear manner in the direction of extent of the roller supports 13, 14. The roller supports 13, 14 are connected to the cam 17.

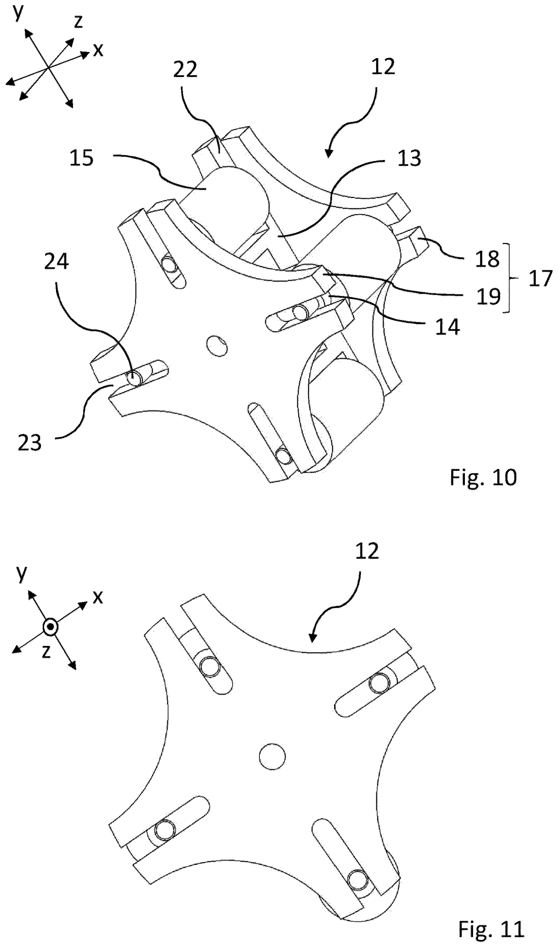

Fig. 10 shows a schematic depiction of an alternative rotor 12 and Fig. 11 shows a view of the rotor 12 from Fig. 10. In contrast to the rotor 12 from Fig. 9, the cam 17 is now configured as two pieces. The cam 17 comprises two cam members 18, 19. The cam members 18, 19 are formed in a cross-shaped manner. The cam members 18, 19 are arranged parallel to one another. The roller supports 13, 14 are arranged displaceably between the cam members 18, 19.

A first cam member 18 has four slots 22. Two slots 22 situated opposite one another extend in the y-z plane. Two of the slots 22 extend in the x-z plane. A second cam member 19 has four slots 23. Two slots 23 situated opposite one another extend in the y-z plane. Two of the slots 23 extend in the x-z plane. Alternatively, the slots 22, 23 can also be designed as cavities, recesses or elongate holes.

Two of the rollers 15 are rotatably mounted on the first roller support 13 and in the slots 22 by means of protruding shafts or pins 24. Two of the further rollers 15 are rotatably mounted on the second roller support 14 and in the slots 23 by means of protruding shafts or pins 24.

In the alternative embodiment of the rotor 12 too, the roller supports 13, 14 and the rollers 15 are set up to be displaced relative to one another. A displacement takes place in a linear manner in the direction of extent of the roller supports 13, 14. The roller supports 13, 14 are connected to the cam 17, preferably in a spring-free manner.

Fig. 12 shows a schematic depiction of a linear peristaltic pump 1. The linear peristaltic pump 1 comprises a device 10, as is shown inter alias in Fig. 1. The linear peristaltic pump 1 furthermore comprises a rotor 12, as is shown inter alias in Fig. 9. The rotor 12 is inserted into the device 10 or arranged in it.

The first roller support 13 and the second roller support 14 are set up to be displaced relative to the roller track 100. The rotor 12 is preferably dimensioned such that the rollers 15 contact the track 100. The linear peristaltic pump 1 furthermore comprises a drive 25 for driving the rotor 12. The drive 25 is connected to the rotor 12.

In the linear peristaltic pump 1, a tube 40 is laid into the groove 26. The groove 26 runs, in sections, parallel to the first subregion 102 from Fig. 1. To secure the tube 40, the device 10 has a closure 30. The closure 30 is rotatably mounted on the device by means of a bolt 32, wherein the side facing the tube 40 provides a central opposed running surface 31 for receiving the tube 40. The bolt is introduced into the abovementioned receptacle 26 and the closure 30.

As a result of a use of the linear peristaltic pump 1 with the device 10, having the defined roller track, it is possible to squeeze, clamp, occlude, preferably clamp off, press off or press together or compress, by means of the rotor 12, the flexible tube 40 which is laid into the device 10, in order to convey fluid through the flexible tube 40 in a defined manner. As already mentioned regarding Fig. 1, the tube 40 protrudes into the device 10 through the aperture 27 in the groove 26. When the rotor 12 is activated, the rollers follow the roller track. As is known, in the region of the groove, the tube protrudes towards the inside of the device from the aperture, so that, when the rollers contact the tube, it is squeezed, clamped or occluded, preferably clamped off, pressed off or pressed together.

Fig. 13 shows a further schematic depiction of the linear peristaltic pump 1 from Fig. 12. The closure 30 is laid downwards in the direction of the device in the leaf plane. In other words, the closure 30 is closed. The tube 40 is arranged in the groove. A tube clamp (not shown) is preferably used in order to prevent a displacement of the tube. The tube clamp may be part of the device or the peristaltic pump.

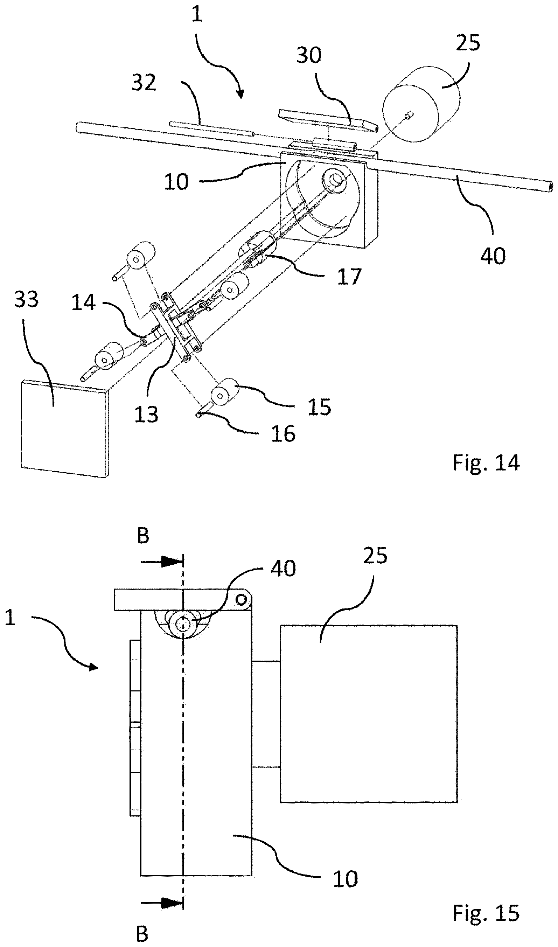

Fig. 14 shows an exploded depiction of parts of the linear peristaltic pump 1. In addition to the component parts already described, the device 10 also comprises a screen 33 which is set up to cover the rotor 12 which is arranged in the device.

Fig. 15 shows a lateral view onto the linear peristaltic pump from Fig. 13. As already mentioned above, the device 10 is set up to squeeze, clamp, occlude, preferably clamp off, press off or press together or compress the tube 40 by means of a roller 15 of a rotor 12 which is rotatably mounted in the device 10. The rotor 12 is preferably dimensioned such that the rollers 15 contact the track 100. In other words, a rotation of the rotor 12 causes a displacement of the roller supports and thus of the rollers 15. It is clear to see that the tube 40, in the region of the aperture, protrudes into the device 10, such that the tube is squeezed by means of the roller(s) of the rotor 12.

The rollers are preferably set up to use the squeezed tube 40 as a track. This can be the case if the tube 40, in the squeezed state, i.e. when at least one roller is in roller contact with the tube 40, still protrudes into the device 10. Particularly preferably, the device and the rotors are dimensioned such that they allow tolerances.

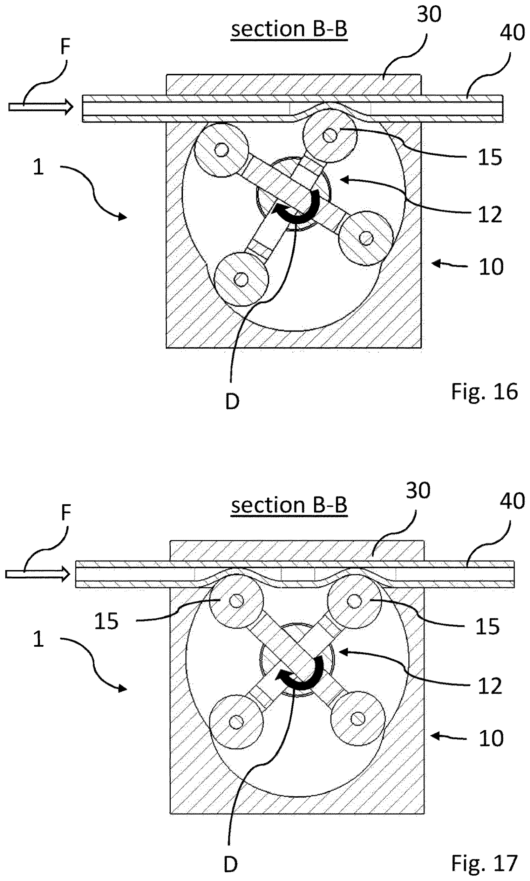

Figs. 16 to 20 depict the rotor 12 in different positions. In Fig. 16 to Fig. 20, a conveying direction for a fluid is specified by F and a direction of rotation of the rotor is specified by D.

Fig. 16 shows the section B-B according to Fig. 15 with a rotor 12 in a first position. One of the rollers 15 is squeezing the tube 40.

Fig. 17 shows the section B-B according to Fig. 15 with the rotor 12 in a second position. Two of the rollers 15 are squeezing the tube 40.

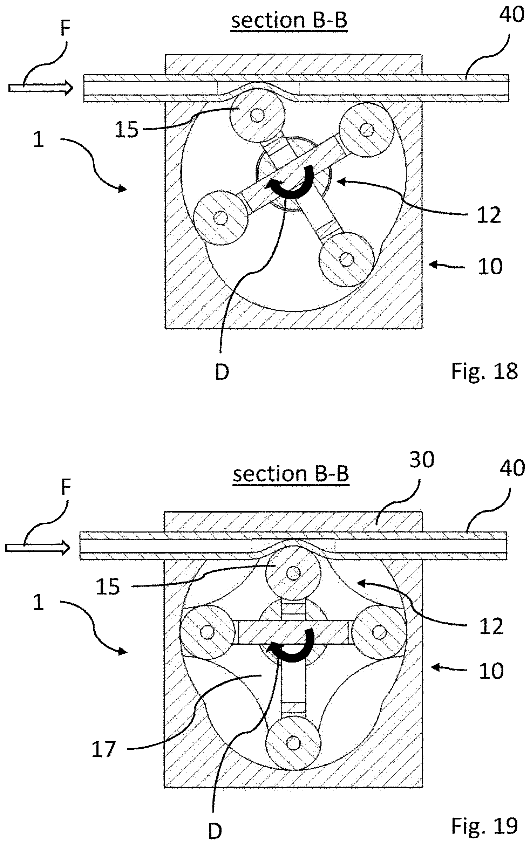

Fig. 18 shows the section B-B according to Fig. 15 with the rotor 12 in a third position. One of the rollers 15 is squeezing the tube 40.

Fig. 19 shows the section B-B according to Fig. 15 with the rotor 12 in a fourth position. One of the rollers 15 is squeezing the tube 40.

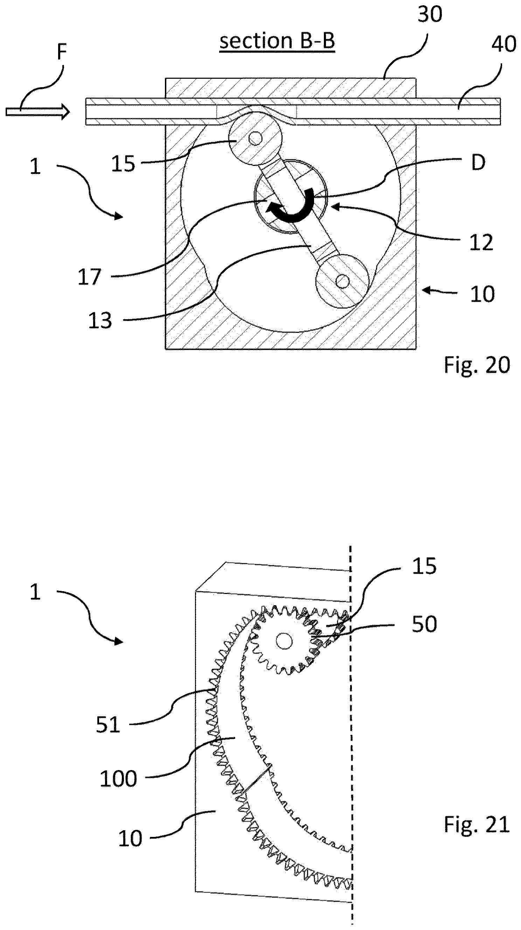

Fig. 20 shows the section B-B according to Fig. 15 with an alternatively configured rotor 12 in a first position. In contrast to the previous embodiments, the rotor 12 comprises only one roller support 13 with two rotatably mounted rollers 15 arranged at the ends. The rotor 12 thus corresponds to the rotor 12 according to Fig. 9, but dispenses with the second roller support 14 and thus with two of the rollers 15.

Fig. 21 shows a partial view of the device from Fig. 1 in an alternative configuration and of a roller of a rotor. The roller track 100 of the device 10 is configured on both sides at the edge as a toothed rack 51 or as a toothing. At least the depicted roller 15 has, on both sides, at the edge, a region configured as a sprocket 50. The toothed rack 5 or the toothing meshes with the sprockets 50 of the roller 15. As a result of this, defined guiding of the rollers 15 along the track 100 is possible.

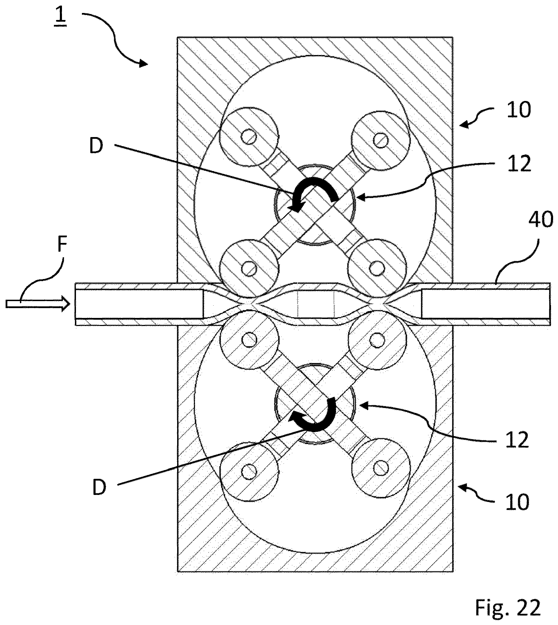

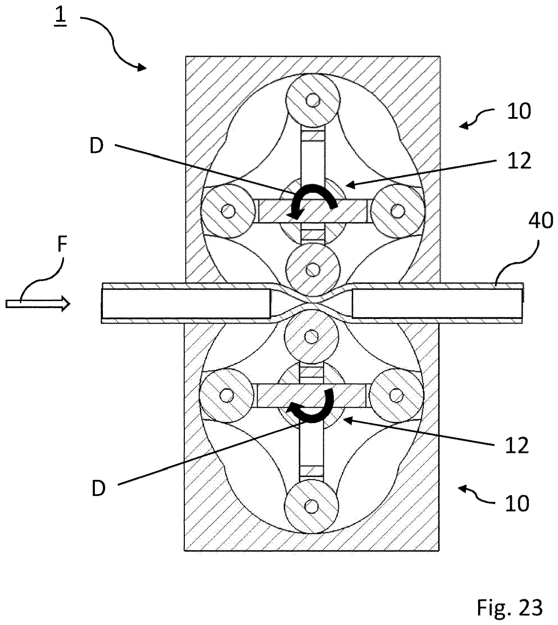

In Fig. 22 and Fig. 23, a conveying direction for a fluid is specified by F and a direction of rotation of the rotor is specified by D.

Fig. 22 shows a section through an alternatively configured linear peristaltic pump 1. The linear peristaltic pump 1 comprises two devices 10 with one rotor 12 each. The devices 10 are arranged relative to one another such that their grooves for accommodating a tube 40 are situated opposite one another. It is thus possible to dispense with the closures, one of which is shown in Figs. 12, 13. The tube 40 is introduced or arranged in the groove of the devices 10 which are arranged below in the leaf plane and into the groove of the devices 10 arranged at the top in the leaf plane. The rotors 12 are located in a first position in order to squeeze the tube 40.

Fig. 23 shows an additional section through the alternatively configured linear peristaltic pump from Fig. 22. Here, the tube 40 is squeezed in the middle on both sides. The rotors 12 are located in a second position in order to squeeze the tube 40 in the middle.

Alongside the device, a rotor and the linear peristaltic pump are also provided. All component parts have a simple structure. All component parts can be manufactured inexpensively. The multipart structure then allows the components to be exchanged without difficulty in the event of damage.

From the foregoing, it will be observed that numerous variations and modifications may be effected without departing from the scope of the invention. It is to be understood that no limitation with respect to the specific device or method described herein is intended or should be inferred. It is, of course, intended to cover all such modifications as fall within the scope of the invention.

### List of reference numbers

- 1: peristaltic pump
- 10: device (stator)
- 12: rotor
- 13: first squeezing means support
- 14: second squeezing means support
- 15: roller
- 16: shaft
- 17: cam
- 18: first cam member
- 19: second cam member
- 20: first slot
- 21: second slot
- 22: third slot
- 23: fourth slot
- 24: pin (peg)
- 25: motor
- 26: groove
- 27: aperture
- 28: connector
- 30: closure
- 31: opposed running surface
- 32: bolt
- 33: screen (lid)

- 40: flexible tube
- 50: sprocket
- 51: toothing

- 100: track, guideway or slideway
- 101: circular course
- 102: first subregion
- 103: second subregion
- 104: first section of circular course or first arc
- 105: second section of circular course or second arc

- 110: first spacing
- 111: further spacing on the tube receiving side
- 120: second spacing
- 121: further spacing opposite of the spacing on the tube receiving side
- 130: aperture
- 132: first direction of movement
- 135: first longitudinal direction/longitudinal axis
- 140: bar
- 142: second direction of movement
- 145: second longitudinal direction/longitudinal axis

- Z: direction of rotation
- Y: conveying direction
- L1: length
- L2: length
- M: midpoint
- R: radius
- P102: first point
- P103: second point

## Claims

1. A device (10) for a linear peristaltic pump (1) for conveying a fluid through a flexible tube (40), with a continuously running track or slideway (100), wherein the track or slideway (100) has, in sections, a circular course (101) with a radius (R) and a midpoint (M), wherein the track or slideway (100) further comprises
a) a first subregion (102) with a linear or curved course which runs spaced apart relative to the circular course (101) and has, in the radial direction, a first maximum spacing (110), and
b) a second subregion (103) with a curved course, wherein the second subregion (103) is arranged opposite the first subregion (102), and wherein the second subregion (103) runs spaced apart relative to the circular course (101) and, in the radial direction, has a second maximum spacing (120),
c) wherein the first maximum spacing (100) and the second maximum spacing are of identical size.

2. The device (10) according to claim 1, wherein, the linear or curved courses of the first subregion and the curved course of the second subregion are configured such that in the radial direction, a first length (L1, L11) is a difference between the radius (R) and a first spacing (110, 111) relative to the circular course and wherein, in the radial direction, a second length (L2, L12) is a sum of the radius (R) and a second spacing (120, 121) relative to the circular course.

3. The device (10) according to claim 2, wherein the first length (L1) defines a first point (P102) on the first subregion (102) and the second length (L2) defines a second point (P103) on the second subregion (103) and/or the sum of the first length (L1) and the second length (L2) corresponds to double the radius R.

4. The device (10) according to one of the preceding claims, wherein the first maximum spacing (110) is arranged in the middle of the first subregion (102) and the second maximum spacing (120) is arranged in the middle of the second subregion (102).

5. The device (10) according to one of the preceding claims, wherein two arcs of the circular course (101) are arranged opposite one another and/or
both ends of the first subregion (102) and of the second subregion (103) are connected to one section of the circular course (101) each.

6. The device (10) according to one of the preceding claims, wherein the second subregion (103) is configured to be convex and/or.
wherein the curved course of the first subregion (102)
- is configured to be arched toward the midpoint (M), or
- is configured to be arched away from the midpoint (M).

7. The device (10) according to one of the preceding claims, wherein the device (10) has a groove (26) for accommodating tube (40), wherein the groove (26) has an aperture (27) in the region of the first subregion (102) and/or wherein the device (10) for securing the tube (40) in the device (10) has a closure (30) or a cover.

8. A rotor (12) for a device (10), preferably according to one of the preceding claims, of a linear peristaltic pump (1) for conveying a fluid through a flexible tube (40), having
- at least one first pair of squeezing means having a first and a second squeezing means (15),
- at least one squeezing means support (13, 14) for accommodating the first and second squeezing means (15), and
- at least one cam (17) for guiding and/or accommodating the at least one squeezing means support (13, 14).

9. The rotor (12) according to claim 8, wherein the cam (17) has slots (20, 21), cavities or recesses, wherein the at least one squeezing means support (13, 14) is displaceably arranged in a slot (20, 21), cavity or recess.

10. The rotor (12) according to claim 8, wherein the cam (17) comprises two cam members (18, 19) arranged parallel to one another and spaced apart from one another, wherein the cam members (18, 19) have slots (22, 23), cavities or recesses for guiding the squeezing means (15), and wherein the at least one squeezing means support (13, 14) is displaceably arranged between the cam members (18, 19), wherein optionally a first longitudinal axis (135) of the first squeezing means support (13, 14) and a second longitudinal axis (145) of a second squeezing means support (13, 14) are arranged perpendicular to one another.

11. The rotor (12) according to one of claims 8 to 10, wherein the at least one squeezing means support (13, 14) is set up to be displaced relative to the cam (17) and/or wherein, in the event of two or more squeezing means supports (13, 14), the squeezing means supports (13, 14) are set up to be displaced relative to the cam (17) and/or relative to one another.

12. A linear peristaltic pump (1) comprising at least one device (10) according to one of claims 1 to 7 and a rotor (12) according to one of claims 8 to 11,
wherein the device comprises a continuously running track or slideway (100), wherein the track or slideway (100) has, in sections, a circular course (101) with a radius (R) and a midpoint (M), wherein the track or slideway (100) further comprises
a) a first subregion (102) with a linear or curved course which runs spaced apart relative to the circular course (101) and has, in the radial direction, a first maximum spacing (110), and
b) a second subregion (103) with a curved course, wherein the second subregion (103) is arranged opposite the first subregion (102), and wherein the second subregion (103) runs spaced apart relative to the circular course (101) and, in the radial direction, has a second maximum spacing (120),
c) wherein the first maximum spacing (110) and the second maximum spacings (120) are of identical size.

13. The linear peristaltic pump (1) according to claim 12, wherein, the linear or curved course of the first subregion (102) and the curved course of the second subregion (103) are configured such that in the radial direction, a first length (L1, L11) is a difference between the radius (R) and a first spacing (110, 111) relative to the circular course and wherein, in the radial direction, a second length (L2, L12) is a sum of the radius (R) and a second spacing (120, 121) relative to the circular course.

14. The linear peristaltic pump (1) according to claim 12, wherein the rotor is configured to squeeze by at least one first pair of squeezing means (15), a flexible tube (40) which is arrangable into a groove (26) of the device (10) and which protrudes into the aperture (27) of the groove (26), in order to convey fluid through the flexible tube (40), wherein the at least one first squeezing means support (13, 14) is set up to be displaced relative to the cam and/or relative to one another and /or relative to the track or slideway (100).

15. The linear peristaltic pump (1) according to one of claims 12 to 14, wherein the track or slideway (100) is configured, at the edge, as a toothed rack (51) or as a toothing and the at least one squeezing means (15) has, at the edge, a region configured as a sprocket (50), wherein the toothed rack (51) or the toothing meshes with the sprockets (50) of the squeezing means (15).
